# EUROPEAN PATENT SPECIFICATION

(11) **EP 2 218 447 B1**
(45) Date of publication and mention of the grant of the patent: **19.04.2017**
(21) Application number: 08019306.3
(22) Date of filing: 04.11.2008
(51) Int. Cl.: A61K 9/51, A61K 8/19, A61K 8/29, A61K 8/27, A61Q 19/00, A61K 8/11, A61K 33/38

(54) **Compositions containing lipid micro- or nanoparticles for the enhancement of the dermal action of solid particles**
Zusammensetzungen mit Lipid-Mikro- oder Nanopartikeln für die Verbesserung der Hautwirkung fester Partikel
Compositions contenant des micro ou nanoparticules lipides pour l'amélioration de l'action dermique de particules solides

(43) Date of publication of application: 18.08.2010
(73) Proprietor: PharmaSol GmbH, 12307 Berlin (DE); Dr. Rimpler GmbH, 30900 Wedemark (DE)
(72) Inventor: Keck, Cornelia, Dr., 12161 Berlin (DE); Schwabe, Kay, 30900 Wedemark (DE); Rimpler, Christian, 30900 Wedemark (DE)
(74) Representative: Uexküll & Stolberg

(56) References cited:
- WO-A2-2008/013785
- WO-A2-2008/089822
- US-A- 5 188 837
- US-B1- 6 814 959
- CENGIZ E ET AL: "Sunblocking efficiency of various TiO(2)-loaded solid lipid nanoparticle formulations(1)." INTERNATIONAL JOURNAL OF COSMETIC SCIENCE OCT 2006, vol. 28, no. 5, October 2006 (2006-10), pages 371-378, XP002550167 ISSN: 1468-2494
- MULLER R H ET AL: "Solid lipid nanoparticles (SLN) for controlled drug delivery - a review of the state of the art" EUROPEAN JOURNAL OF PHARMACEUTICS AND BIOPHARMACEUTICS, ELSEVIER SCIENCE PUBLISHERS B.V., AMSTERDAM, NL, vol. 50, no. 1, 3 July 2000 (2000-07-03), pages 161-177, XP004257186 ISSN: 0939-6411
- KECK C M ET AL: "A new concept for the treatment of atopic dermatitis: Silver-nanolipid complex (s", INTERNATIONAL JOURNAL OF PHARMACEUTICS, ELSEVIER BV, NL, vol. 462, no. 1, 27 December 2013 (2013-12-27), pages 44-51, XP028821682, ISSN: 0378-5173, DOI: 10.1016/J.IJPHARM.2013.12.044

## Description

### 1. Background

Major dermal problems are hypersensitivity of the skin, dry skin, atopic appearance of the skin, or severe state skin diseases like atopic dermatitis (in German: Neurodermitis) and psoriasis. These changes in the skin condition are characterized by itching, burning feeling, reddening of the skin and damaged skin surface (e.g. cracks in the skin etc.). There are many dermal formulations (creams, lotions, sprays etc.) on the market either as cosmetics or as pharmaceutical products. Whereas pharmaceutical products treat a skin disease, cosmetic products are supportive in normalization of a skin condition or being supportive in addition to a pharmaceutical/medical treatment. Cosmetics contribute to improve the situation of the cells of the skin without having a therapeutic action.

The skin condition can even worsen in case dermal formulations are applied which contain preservatives. The preservative principle is to interact with the cell membranes of microbes, to damage the cell membrane which leads to the death of the cells. Of course, the preservatives also interact with the cells of the human skin which can lead to a more pronounced appearance of skin damage, e.g. reddening and itching. They have also allergic potential, well documented in the literature [Lee, E., An, S., Choi, D., Moon, S., Chang, I., 2007. Comparison of objective and sensory skin irritations of several cosmetic preservatives. Contact Dermatitis, 56, 131 - 136; Steinberg, D., 2006. Preservatives for Cosmetics, Allured Publishing Corporation, Carol Stream] Therefore there are increasing attempts to replace the "traditional preservatives" registered in the regulatory lists as preservatives (e.g. ANNEX VI - List of preservatives which cosmetic products may contain - Part I (Cosmetics Directive 76/768/EEC)) by compounds having no official preservative status but having anti-microbial action. Examples are propylene glycol, pentylene glycol, ethanol or natural plant extracts (e.g. grapefruit seed extract). When using these compounds the products can be labelled as preservative-free. However, also these anti-microbial compounds can exhibit undesired side effects, especially plant extracts bearing the risk of allergic reactions.

### 2. State of the art

An interesting alternative is the use of silver, added to the formulations in particulate form. It is well known in pharmaceutical technology that silver ions possess an antimicrobial activity (so called oligodynamic effect [List, P.H., 1982. Arzneiformenlehre: Lehrbuch für Pharmazeuten, Wissenschaftliche Verlagsgesellschaft, Stuttgart]. The first who described the anti-microbial effect of silver was Carl von Nägeli [von Nägeli, C.W., 1893. Über oligodynamische Erscheinungen in lebenden Zellen. Neue Denkschriften der schweizerischen naturforschenden Gesellschaft, pp. 1-51]. Commercially available are various silver products to be added to creams or lotions to exploit the antibacterial action of the silver. These products contain solid silver particles with a size in the micrometer range (e.g. average size about 10 µm, product MicroSilver BG, BioEpiderm GmbH Nürnberg / Germany) [Bechert, T., Wagener, M., Steinrücke, P., 2003. Körperpflegemittel mit Silber und Zink. EP 1 897 593 A2]. Silver particles are also available with a size in the nanometer range. The silver in the nanometer range has the disadvantage of the black colour. Therefore in these investigations microsilver was used, being whitish. Typical concentrations are between 0.2% and 1.5%. The action is attributed to the very low concentration of silver ions dissolved from the silver particles [Renner, H., 1982. Silber, Silber-Verbindungen und Silber-Legierungen. Bartholomé, E., Biekert, E., et al. (Ed.), Ullmanns Encyklopädie der technischen Chemie, Chemie Verlag, Weinheim, New York]. The silver particles remain on the skin, there is practically no penetration of silver ions into the skin. Even when applying a cream with 1.5% silver, the penetration into the skin is less than 0.001% of the applied dose (in vitro, dose 20 mg cream per cm²). The skin particles remain on the skin, pronounced in the skin folds. Within their antimicrobial action the silver ions are also effective against Staphylococcus aureus, a major skin pathogen in many skin diseases.

Atopic dermatitis is an inflammatory, in most cases chronic skin disease, having a multifactorial pathogenesis. Causal therapy is not possible, therefore only symptomatic therapy is performed using e.g. glucocorticoids, immune-suppressive drugs, antiseptics, very often in combination with a "dermo-cosmetic" skin care [Abels, C., Proschke, E., 2006. Therapie des atopischen Ekzems. Hautarzt 57, 711-725]. The term dermo-cosmetic includes that more sophisticated cosmetic products are required for the basic skin care of these patients, considering the type of atopic skin and especially the higher sensitivity, e.g. towards preservatives used in the products. Or in other words: a simple petrolatum / vaselinum cream is not considered as a sensible formulation for restoring the damaged barrier function of the skin in such diseases.

Apart from the anti-microbial action, it was found that the silver particles improve wound heeling and acute symptoms (e.g. redness, itchiness) in diseases like atopic dermatitis. The efficiency of the German commercial product Multilind^{®} MicroSilver Cream was investigated for treating atopic eczema in a human study [Ekanayake-Mudiyanselage, S., Balk, A., Schoder, V., Hansen, P., Wigger-Alberti, W., Wilhelm, K.-P., 2007. Anwendungsbeobachtung mit einem topischen silberhaltigen Pflegeprodukt (Multilind® MikroSilber Creme) zur Überprüfung der Wirksamkeit, verträglichkeit und kosmetischen Akzeptanz beim atopischen Ekzem. Kosmetische Medizin 28, 291-295]. The cream contained 0.3% microsilver. Application was performed twice daily, evaluation of the skin took place on day 8 and day 15. Result was a significant improvement and reduction of the local SCORAD (SCORAD - Severity scoring of atopic dermatitis). Tolerability was evaluated with "very good", treatment efficiency with "good" to "very good". However, it should be noted that the atopic dermatitis was a mild one, not a severe one.

It is described that formulations which contain additionally to the silver particles soluble silver salts, particulate zinc oxide and chitosan or chitosan derivatives possess a synergistic biocide effect [Schmid, H., 2007. Nanopartikulaeres Silber enthaltende biozide Zusammensetzung. DE 102005041005 A1 UPAB: 20070510 NOVELTY Patentblatt, Viol.127 (2007), No.09] H. Schmid uses nanosilver, size below 500 nm. By using the silver in its nanosize, it is very effective and the concentrations in products according to this invention are below 500 ppm, especially below 100 ppm and preferred between 1 and 100 ppm.

The anti-microbial effect of silver and zinc ions is also exploited in consumer care products such as tooth paste (WO 00/06208 A1) based on ion exchange, in which a part of the exchangeable ions are silver and zinc ions. Zinc ions act synergistically with silver ions (DE102007001466A1). Copper ions are used in wine making industry against moulds. Therefore it makes sense to make combinations of the three ions silver, zinc and copper.

Repair of the skin barrier function is described for lipid nanoparticles in cosmetic products [Muller, R.H., Rimpler, C., 2007. A New Dimension in Cosmetic Products by Nanostructured Lipid Carriers (NLC) Technology. EuroCosmetics, 3; Müller, R.H., Petersen, R.D., Hommoss, A., Pardeike, J., 2007. Nanostructured lipid carriers (NLC) in cosmetic dermal products. Adv Drug Deliv Rev, 59, 522-530]. Nanoemulsions are composed of fine droplets of a liquid lipid (oil), in contrast lipid nanoparticles are particles in the nanometer range possessing a solid matrix. This matrix is composed of lipids being solid at body temperature. In case of solid lipid nanoparticles (SLN) [Lucks, J.S., Müller, R.H., 1996. Medication vehicles made of solid lipid particle (solid lipid Nanospheres - SLN). EP0000605497] the matrix consists of a solid lipid only (e.g. glycerides of behenic acid, bees wax, carnauba wax etc.). In case of nanostructured lipid carriers (NLC) the matrix consists of a blend of solid lipids with liquid lipids (oils), but this blend being also solid at body temperature [Müller, R.H., Jenning, V., Mäder, K., Lippacher, A., 2000. Lipid particles on the basis of mixtures of liquid and solid lipids and the method for producing same. PCT/EP2000/004112] The lipid nanoparticles can be used unloaded consisting of lipids only, or loaded with cosmetic actives such as vitamin E, coenzyme Q10 or drugs such as glucocorticoids, cyclosporine, etc. Detailed reviews give an overview of the special features of lipid nanoparticles (Müller, R.H., Mehnert, W., Lucks, J. S., Schwarz, C., zur Mühlen, A., Weyhers, H., Freitas, C., Rühl, D., 1995. Solid Lipid Nanoparticles (SLN) - An Alternative Colloidal Carrier System for Controlled Drug Delivery. Eur. J. Pharm. Biopharm., 41, 62-69; Müller, R.H., Mäder, K., Gohla, S., 2000. Solid lipid nanoparticles (SLN) for controlled drug delivery - a review of the state of the art. Eur. J. Pharm. Biopharm., 50, 161-177; Müller, R.H., Petersen, R.D., Hommoss, A., Pardeike, J., 2007. Nanostructured lipid carriers (NLC) in cosmetic dermal products. Adv. Drug Delivery. Rev., 59, 522-530; Pardeike, J., Hommoss, A., Muller, R.H., 2008. Lipid nanoparticles (SLN, NLC) in cosmetic, pharmaceutical dermal products. Int. J. Pharm., in press, doi:10.1016/j.ijpharm. 2008.1010.1003]. Lipid nanoparticles were also found to enhance the hydration of the skin due to their occlusive effect. Based on this the lipid nanoparticles are a suitable ingredient for all skin conditions (cosmetic or medical) associated with a disturbed barrier function due to damage of the lipid film.

To sum up: There are unmet needs to treat skin conditions like hypersensitivity, atopic dermatitis and psoriasis. The dermal formulations should be as little irritating as possible to the a priori sensitive skin, that means should be preservative-free. In addition, the "dermo-cosmetic" supportive skin care should be as efficient as possible to prolong the symptom-free intervals of the disease and to shorten the duration of acute exacerbation. Shortening the acute phase reduces treatment time with glucocorticoids and minimizes related side effects (e.g. skin atrophy). Ideally the dermo-cosmetic formulation should contain compounds which are not a pharmaceutical drug but promote the normalization of the cell function.

### 3. Brief description of the invention

To meet the unmet needs described above, the invention provides a composition according to claim 1. Preferred embodiment are subject of the dependent claims As specific and preferred embodiments a cosmetic cream (example 2) and a cosmetic emulsion (example 3) were prepared. They were prepared by combining the silver particles and lipid nanoparticles to formulate an improved dermo-cosmetic product. Lipid nanoparticles were prepared as described in example 1 and added during the production process of the cream and the emulsion, respectively. The use of silver particles allows production of a preservative-free product. In addition, the anti-microbial silver supports the healing process of irritated skin or skin with mild atopic eczema, especially in combination with a medical treatment, as described by S. Ekanayake et al. (Kosmetische Medizin, 6, 291-295 (2007)). The skin care effect is further supported by the lipid nanoparticles, which are described to adhere to the stratum corneum and might contribute to the repair of a damaged skin lipid film.

The efficiency of the cream was tested by 20 volunteers, 2 examples are given. Surprisingly the treatment efficiency was beyond what could be theoretically expected based on the published literature. Ekanayake et al. describe a good treatment efficiency with silver particles for mild atopic eczema. Volunteers treated themselves with the cream of the invention who were in therapy with glucocorticoids without showing any improvement of the skin condition (examples 6 and 7). In one case the glucocorticoid treatment even worsened the symptoms (example 6). The application of the invented cream was so efficient in skin normalisation that it could replace medical treatment with glucocortocoids. Treatment efficiency is documented in examples.6 and 7.

Surprisingly, obviously a synergistic effect was found for silver particles in conjunction with lipid nanoparticles. Synergistic effects are described in the literature for e.g. silver and zinc oxide particles, or with copper particles. This synergism can be logically explained by the release of metal ions from all these particles interacting e.g. with the microbes on the skin, or playing a role in anti-oxidation (e.g. silver). However, nothing like this can occur with the lipid nanoparticles, therefore this effect was not predictable, especially not in the observed extent.

Based on the findings it can be concluded:
1. Optimal silver action is achieved by the combination of silver particles with lipid nanoparticles (SLN or NLC).
2. Microsilver and lipid nanoparticles seem to act synergistically as seen by the pronounced in vivo effects on the skin.
3. Higher silver concentrations, being active at the beginning of atopic dermatitis (e.g. 1.5%) can be replaced by 10 times and more lower silver concentrations, when used in combination with the lipid nanoparticles (concentration in emulsion and cream: 0.10-0.15%).
4. The action of silver ions against Staphylococcus aureaus seems to be increased, with simultaneously even less action against the normal skin Staphylococcus Epi, because silver concentrations are reduced and thus normal cells are less affected.
5. Lipid nanoparticles in combination with microsilver normalize the skin condition by repairing the damaged lipid barrier and simultaneously the anti-inflammatory, anti-oxidative action. Both promote the restoration of the normal physiology of the skin.

### 4. Detailed description of the invention

The silver particles act to improve the skin condition in mild cases of irritation (reddening) and mild atopic eczema/dermatitis. These skin conditions are accompanied with a distorted skin barrier, e.g. the state of the lipid film on the surface of the stratum corneum. The Lipid nanoparticles repair a damaged lipid film of the skin, or the re-inforce a thin lipid film. Restoration of the lipid barrier is one prerequisite for normalization of the physiological situation of the cells of the epidermis. The invention provides a combination of silver particles and lipid nanoparticles. For the repair effect, both lipid nanoparticles SLN and NLC can be used. It is a pure mechanistic effect of covering a surface by a highly adhesive nanomaterial, in this case the lipid nanoparticles.

The adhesiveness of a material is a function of size. An example from daily live is sugar. Rocky candy sugar does not stick at all to bakery, crystalline sugar sticks quite well, iced sugar adheres nicely to bakery. The same is valid for lipid nanoparticles, being comparable to the iced sugar. In general, the adhesiveness increases with decreasing size. Therefore theoretically one should use e.g. nanoparticles with a size of a few ten of nanometers. However, the smaller the particles, the more difficult and expensive are the particles to produce. That means one has to compromise between costs and adhesiveness. Lipid nanoparticles are per definition below 1,000 nm. In studies it was found that particles below 500 nm are more adhesive than larger nanoparticles. Preferably the lipid nanoparticles should be below 200 nm and be preferred in the range of 20-100 nm (mean sizes determined by photon correlation spectroscopy - PCS).

However, the use of lipid microparticles is also sensible, especially when a prolonged release of an incorporated cosmetic or pharmaceutical active is desired. Release is slower with increasing size of the particles, therefore microparticles are more suited for this than nanoparticles.

Microparticles can rather be compared with the crystalline sugar from daily life. In addition it needs to be considered, that too large microparticles can be sensed when applying a product to the skin. Therefore, the size should be as large as possible, to prolong release as long as possible. On the other hand the size should be as small as possible, to minimize sensing effects during application. Normally particles with a size of about 40 µm and larger are sensed during application to the skin. Considering these aspects, as a compromise the lipid microparticles should possess a size below 200 µm, especially below 50 µm, preferentially 20 µm and preferred being in the range 1-10 µm (= higher adhesiveness). The silver particles in the dermal product can be made from chemical compounds containing silver, in particular silver oxide. The particles are available in different sizes and physical forms. There are solid particles available in nanometer and in micrometer sizes. The nanosilver has the disadvantage of the blackening of products, being less aesthetically appealing. In addition, the particles can be sponge-like, like the silver particles used for the preparation of the cream and the lotion from examples 2 and 3. Sponge like particles have the advantage of a more whitish colour, in addition - due to the large surface area - dissolution of silver ions is fast when applying the product to the skin and getting in touch (diluted) with body fluids (e.g. water on the surface of the skin).

When using silver particles in the micrometer range, to minimize sensing during application to the skin, they should have a mean diameter of 1 to 100 µm, especially 1 to 40 µm (no or little sensing) and preferentially 1 to 20 µm (faster dissolution due too large surface, stronger adhesion to the skin).

The silver particles are contained in the water phase of a cream or lotion. They can also be dispersed in the water phase of a gel or a fluid (= suspension).

Certain chemical compounds of silver further promote the action. Therefore formulations can be made which contain additionally soluble salts of silver (e.g. silver nitrate, silver carbonate, silver sulphate, silver acetate, silver benzoate, silver lactate), organic compounds of silver (e.g. silver acetylacetonate, silver neodecanoate, silver ethylenediamin-tetraacetate) In addition it was found that certain polymers are supportive in the biocide action, especially chitosan and chitosan derivatives. Of course it makes sense to use even mixtures thereof.

For application to the skin or other parts of the body, the lipid nanoparticles, silver ions and potentially other compounds (e.g. silver salts, polymers such as chitosan) can be incorporated in a so called matrix to be applied or administered to the body. In case of dermal application this can be e.g. a gel, a cream or a lotion. In case of applying it to body cavities a suspension might be more suited. In any case, the final formulation with these compounds (product) consists of a matrix, which contains the compositions according to claims 1 to 8. The matrix can contain only lipid nanoparticles with silver particles; or: lipid nanoparticles with silver and zinc particles. The ratios can vary and need to be optimised according to the skin condition to be normalised or optimised (cosmetic products) or the disease to be treated (pharmaceutical products).

Basically the formulation of the matrix can be a gel (e.g. polyacrylate, Xanthan, cellulose derivatives), a fluid suspensions of lipid nanoparticles and silver particles), an oil-in-water cream or water-in-oil cream, or an ointment, including the excipients used in these formulations being known from the text books.

Ideally the products should not contain surfactants (e.g. sodium dodecyl sulphate (SDS), polysorbates etc.). This can be achieved by replacing the classical surfactants by sterically stabilizing polymers such as poloxamers (block polymers of polyethylene oxide and propylene oxide, e.g. available from BASF/Germany or ICI/UK). Sterically stabilizing polymers act very often simultaneously as viscosity enhancers, and are therefore also suited to produce more viscous fluids. Further examples are polyvinyl alcohol (PVA), Xanthan gum, polyacrylates and gelatines.

The products containing silver particles are anti-microbial, therefore they do not need preservatives as defined by the regulatory bodies in the official lists of preservatives (that means the products are preservative-free according to the legal regulations). The risk of contamination during usage by the consumer can be further minimized by using airless packaging. In addition a small drop of silver can be placed at the outlet, to release additional silver ions for preservation (as done e.g. in pharmaceutical eye drop packaging). The formulations according to the invention are preservative-free, reducing further their potential to irritate the skin.

In the present invention the silver ions released from the silver particles possess an anti-oxidative action. This anti-oxidative action is also being held responsible for the effects on the skin condition.

Active compounds can only be incorporated as nanocrystals or microcrystals in case their concentration in the product is above their solubility in the formulation. To avoid Ostwald ripening, the solubility should be below 5%. For example in the case of caffeine, the solubility in water is about up to approximately 4% at room temperature, depending on the pH. Preferentially suitable for incorporation as crystals are compounds with a solubility in water below 1% (appr. 10 mg/ml), better suited are compounds with a solubility below 1 mg/ml, especially 0.1 mg/ml (100 µg/ml), or even better below 10-50 µg/ml.

The consumer care, cosmetic or pharmaceutical formulations can further be increased in their efficiency by incorporating additional active substances in the formulation. These are active substances for consumer care, cosmetic use, pharmaceutical use, medical use, alone or in mixtures thereof.

Actives for incorporation into the formulations according to the invention are primary metabolites of plants such as alkaloids (e.g. caffeine), secondary metabolites of plants (e.g. carotinoids (e.g. β-carotene, lutein), phytosterols (e.g β-sitosterine), phytooestrogens (e.g. genistein), phenolic acids (e.g. ferulic acid), the various tetrahydrocannabinols (THC), and flavonoids, terpens (especially menthol, silymarin and camphor).

Examples for cosmetic actives are vitamin E, Tocotrienol, vitamin A, coenzyme Q10, Argireline (INCI: acetyl hexapeptide-8), derivatives of Argireline, hyaluronic acid, menthol, sage extract, alone or in mixtures thereof.

Also pharmaceutical actives can be incorporated into the formulations of the invention. The mode of incorporation can vary, e.g. incorporation into the lipid particles, dissolution of the active in the oil of the cream or emulsion matrix, addition of the actives in form of nanocrystals or microcrystals etc. Examples for pharmaceutical actives are immune suppressive drugs (e.g. cyclosporine, tacrolimus, sirolimus), glucocorticoids (e.g. dehydrocortison, prednicarbate, dexamethasone, hydrocortison etc.), anti-virals, antimycotics (e.g. Amphotericin B, bifonazole, clotrimazole, itraconazole), antibiotics (e.g. tetracycline base), local anaesthetics (e.g. bupivacaine, tetracaine base), analgesics (e.g. diclofenac, ibuprofen, acetylsalicylic acid), anti-inflammatory drugs, cytotoxics (e.g. paclitaxel, 5-fluorouracil), aescin, minoxidil, hormones (e.g. estradiol, progesterone), caffeine, tretinoin, Sphingosin 1-Phosphat (S1P), alone or in mixtures thereof.

Products according to the invention are for human use, especially for but not limited to consumer care, cosmetic use, pharmaceutical use and medical use, including in connection with cosmetic or medical devices (e.g. laser abrasion equipment for beauty treatment). Especially after laser treatment of the skin, formulations are required acting aseptically without irritating the skin and at the same time supporting the regeneration process. The products of the invention are also suitable for veterinary use, especially for pets and livestock.

Products according to the invention can be used for application to different parts of the body, for example but not limited to the skin, mucosal surfaces, hair, nails and body cavities. The products need to be formulated adequately for optimised performance. These product formulations include, emulsions, creams, lotions, gels, ointments, skin protective creams or skin protective ointments.

The concentration range of microsilver particles is typically but not limited to 0.00001% to 10%, or reduced to 5% and below, preferably below 1% and with optimized concentrations being in the range 0.00001% to 0.15% (e.g. example 2).

Examples of formulations are given as example 2 and 3. Important is that the lipid particles are preferentially present in a non-aggregated state.

### Examples

### Example 1:

### Preparation of Lipid Nanoparticles:

Lipid nanoparticles were prepared by high pressure homogenization. The composition of the dispersion of lipid nanoparticles was: Cutina CP 18,0% , hemp seed oil 12,0%, Tego Care 450, Inutec SP 1, distilled water 67.5% (weight %). The lipid phase including Tego Care 450 and separately the water phase containing the stabilizer Inutec SP were heated to 80° C. Then the lipid phase was dispersed in the aqueous phase under stirring (3,800 rounds per minute, anchor stirrer, 14 minutes). The obtained coarse emulsion was subsequently homogenized at 800 bar, 2 homogenization cycles (Nanomix, Ekato systems, Germany) and cooled under slight stirring to 25° C. The PCS particle size was 225 nm (PCS - photon correlation spectroscopy, Zetasizer Nano ZS, Malvern Instr., United Kingdom). Laser diffractometry diameters were diameter 50% 0.249 µm, diameter 90% 0.468 µm, diameter 95% 0.541 µm and diameter 99% 0.667 µm (Malvern Mastersizer 2000, Malvern Instruments, UK; diameters based on volume size distribution). The zeta potential was -49 mV, measured in distilled water with a conductivity of 50 µS/cm, adjusted by addition of NaCl solution (Zetasizer Nano ZS, Malvern Instr.).

### Example 2:

### Preparation of microsilver cream:

The composition of the cream is given in the table below:

**Table 1:**

| **excipient** | **weight %** | **quantities required for 180 kg batch (kg)** |
|---|---|---|
| **Phase I** | | |
| Squalan | 5.00 | 9.000 |
| Controx KS | 0.15 | 0.270 |
| Hemp seed oil | 4.00 | 7.200 |
| Ubuntu Ximeria | 4,00 | 7.200 |
| Prolipid 141 | 7.00 | 12.600 |
| Lanette O | 3.00 | 5.400 |
| Inutec SP1 | 0.50 | 0.900 |
| Myritol 312 | 3.00 | 5.400 |

| **Phase II** | | |
|---|---|---|
| Water | 44.40 | 79.920 |
| Propylene glycol USP | 3.00 | 5.400 |
| Natrlquest | 0.10 | 0.180 |
| Sorbidex NC 16205 | 3.00 | 5.400 |
| Keltrol BT | 0.30 | 0.540 |
| Kelcogel F | 0.20 | 0.360 |
| Avicell PC | 0.20 | 0.360 |

| **Phase III** | | |
|---|---|---|
| Defensil | 5.00 | 9.000 |
| NLC suspension (example 1) | 10.00 | 18.000 |
| | 2.0 | 3.600 |
| HerbEx Kudzu | 3.0 | 5.400 |
| Antarcticine | | |

| **Phase I** | | |
|---|---|---|
| Caprylyl glycol | 2.0 | 3.600 |
| Microsilver | 0.15 | 0.270 |

The phase I is heated to about 80° C. Phase I is placed in a blender and also heated to 80° C. In the next step phase II is added to phase I under stirring, after appr. 15 min of stirring the cream is cooled. After cooling the phases III and IV are added under stirring and stirred until the cream appears macroscopically homogenous. Blender and stirrer type used depends on the batch size. The droplet size of the bulk population of the droplets in the cream is below 5 µm (light microscopy).

The composition of the cream according to INCI nomenclature is:
Aqua, Cannabis Sativa (Hemp) Seed Oil, Squalane, Octyldodecanol, Ximenia Americana Seed Oil, Caprylic/Capric Triglyceride, Cetearyl Alcohol, Propylene Glycol, Sorbitol, Caprylyl Glycol, Glyceryl Stearate, Behenyl Alcohol, Cetyl Palmitate, Palmitic Acid, CI 77820 (Silver), Pseudoalteromonas Ferment Extract, Helianthus Annuus Seed Oil Unsaponifiables, Cardiospermum Halicacabum Extract, Echium Plantagineum Seed Oil, Tocopherol, Pueraria Lobata Root Extract, Stearic Acid, Aspartic Acid, Lecithin, Polyglyceryl-3 Methylglucose Distearate, Inulin Lauryl Carbamate, Myristyl Alcohol, Lauryl Alcohol, Cetyl Alcohol, Xanthan Gum, Gellan Gum, Cellulose, Cellulose Gum, Hydrogenated Palm Glycerides Citrate, Sodium Hydroxide, Sodium Chloride, Trisodium Ethylenediamine Disuccinate, Butylene Glycol

### Example 3:

### Preparation of microsilver emulsion:

Production was performed as described in example 2, the droplet size of the bulk population of the droplets in the cream is below 5 µm (light microscopy). The composition of the emulsion is given in the table below:

**Table 2:**

| **excipient** | **weight %** | **quantities required for 180 kg batch (kg)** |
|---|---|---|
| **Phase I** | | |
| Squalan | 4.00 | 5.600 |
| Controx KS | 0.15 | 0.210 |
| Hemp seed oil | 3.00 | 4.200 |
| Ubuntu Ximeria | 4.00 | 5.600 |
| Prolipid 141 | 5.00 | 7.000 |
| Lanette O | 2.00 | 2.800 |
| Inutec SP1 | 0.50 | 0.700 |
| Myritol 312 | 4.00 | 5.600 |

| **Phase II** | | |
|---|---|---|
| Water | 58.55 | 81.970 |
| Propylene glycol USP | 3.00 | 4.200 |
| Natrlquest | 0.10 | 0.140 |
| Sorbidex NC 16205 | 3.00 | 4.200 |
| Keltrol BT | 0.20 | 0.280 |
| Kelcogel F | 0.20 | 0.280 |
| Avicell PC | 0.20 | 0.280 |

| **Phase III** | | |
|---|---|---|
| Defensil | 3.00 | 4.200 |
| NLC suspension (example 1) | 5.00 | 7.000 |
| | 2.00 | 2.800 |
| HerbEx kudzu | | |

| **Phase IV** | | |
|---|---|---|
| Caprylyl glycol | 2.00 | 2.800 |
| Microsilver | 0.10 | 0.140 |

The composition of the emulsion according to INCI nomenclature is:
Aqua, Squalane, Ximenia Americana Seed Oil, Caprylic/Capric Triglyceride, Cannabis Sativa (Hemp) Seed Oil, Propylene Glycol, Octyldodecanol, Sorbitol, Caprylyl Glycol, Cetearyl Alcohol, Glyceryl Stearate, Behenyl Alcohol, Helianthus Annuus Seed Oil Unsaponifiables, Echium Plantagineum Seed Oil, Pueraria Lobata Root Extract, Cardiospermum Halicacabum Extract, Tocopherol, CI 77820 (Silver), Aspartic Acid, Lecithin, Cetyl Palmitate, Palmitic Acid, Stearic Acid, Polyglyceryl-3 Methylglucose Distearate, Inulin Lauryl Carbamate, Cetyl Alcohol, Myristyl Alcohol, Lauryl Alcohol, Gellan Gum, Xanthan Gum, Cellulose, Cellulose Gum, Hydrogenated Palm Glycerides Citrate, Sodium Hydroxide, Sodium Chloride, Trisodium Ethylenediamine Disuccinate, Butylene Glycol

### Example 4:

### Assessment of non-irritancy of the lotion by human patch test:

The epicutaneous patch test allows the assessment of primary skin irritation or lack of skin irritation potential of finished cosmetic products. The study was conducted in accordance with the guidelines by COLIPA (A. P. Walker et al., Test guidelines for assessment of skin compatibility of cosmetic finished products in man, Food and Chemical Toxicology 34, 1996, 651-660). As human study it was conducted in accordance with the Declaration of Helsinki (1964) and its subsequent revisions.

Experiments were carried out on 50 volunteers (22 normally healthy subjects, 7 eczema patients, 3 allergy patients, 18 subjects with sensitive skin) between the ages of 22 to 61. Sex distribution was not standardized. The volunteers were clearly informed about all details of the study and gave their written consent. Inclusion criteria were a) informed volunteers and b) age 18 and above. Exclusion criteria were pregnant or lactating women, blemishes or marks (tattoos, sunburn) which interfere with scoring and any skin disease that may interfere with the aim of the study.

Irritancy was assessed by performing a patch test as described in (J.E. Wahlberg, Patch Testing, in: Textbook of Contact Dermatitis, editors: R. J. G. Rycroft, T. Menné, P. J. Frosch and J. P. Lepoittevin, Springer-Verlag, Berlin, 435-468, 2001). The product was applied undiluted in square test chambers (Haye's Test Chambers: HAL Allergic GmbH, Düsseldorf, Germany) to the backs of the panellists for a period 48 hours. Sodium dodecyl sulphate (SDS) in a concentration of 1% was used as positive control, water was used as negative control.

The treatment sites were assessed for the presence of irritation by a trained evaluator using a 5 point visual scoring scale at 48 hours (30 min after patch removal) and after 72 hours after patch application. The scoring scale was:
Erythema (E): 0 (no E), 1 (slight E), 2 (significant E), 3 (pronounced E), 4 (strong E)
Fissure (F) : 0 (minimal F), 1 (moderate), 2 (significantly perceptible), 3 (pronounced F), 4 (ulceration)
Scaling (Sc): 0 (no sc), 1 (minimal Sc), 2 (moderate Sc), 3 (significant Sc), 4 (closed scale crust)

After 48 hours and after 72 hours SDS caused positive reactions in 11 subjects. The scores for E, F and S were in between 0.06 to 0.22 for the positive control SDS solution. None of the subjects showed any reaction to the test product. The mean values of the scoring were 0.0 for E, F and S, respectively, for the test product lotion and for the negative control water. On the basis of the test results and under the test conditions, the lotion is to be classified as "harmless" as regards the possibility of skin reaction.

Data from cosmetic trial report no. 0837-05, human patch test, 29 September 2008 by Derma Consult, Brunnenstr. 61, 53347 Alfter, Germany (investigators: Dr. med. H. Prieur, Dr. rer. nat. H.-P. Nissen).

### Example 5:

### Assessment of non-irritancy of the cream by human patch test:

The test was performed as described in example 4. The mean values of the scoring were 0.0 for E, F and S, respectively, for the test product cream and for the negative control water. On the basis of the test results and under the test conditions, the cream is to be classified as "harmless" as regards the possibility of skin reaction.

Data from cosmetic trial report no. 0834-01, human patch test, 29 September 2008 by Derma Consult, Brunnenstr. 61, 53347 Alfter, Germany (investigators: Dr. med. H. Prieur, Dr. rer. nat. H.-P. Nissen).

### Example 6:

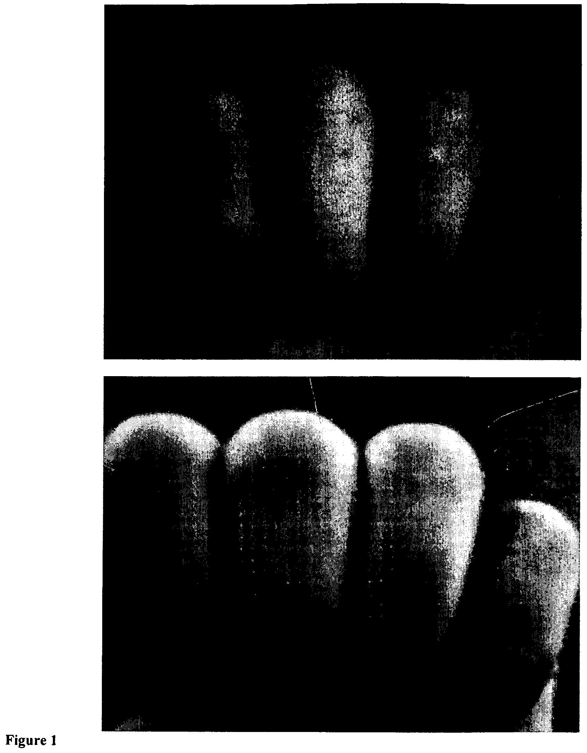

The microsilver cream was applied by a female human volunteer, age 17, twice daily to the fingers. The volunteer was in medical therapy before, applying a corticoid cream twice daily. The cream did not improve the skin condition, it even worsened it. At begin of the study with the microsilver cream, the glucocorticoid treatment was terminated. After about 1 week of treatment, the skin condition improved clearly, but the fingers showed still clear irritation with many pronounced red spots (Fig. 1, upper). Three weeks after treatment, the spots had disappeared, the fingers showed a normal whitish colour (Fig. 1, lower).

### Example 7:

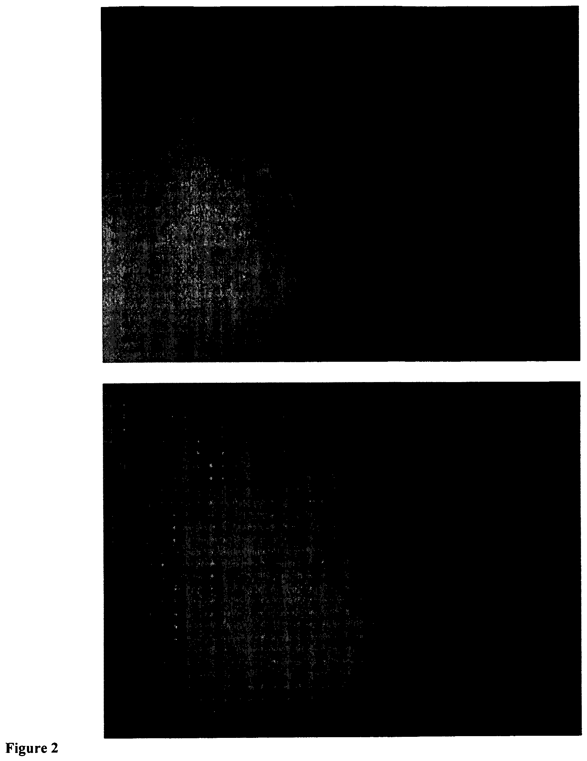

The microsilver cream was applied by a female human volunteer, age 23, body weight 65 kg, to the crook of the arm. The volunteer was in medical treatment applying dehydrocortison cream to the skin. Treatment was terminated at begin of the study. The skin showed pronounced atopic symptoms at the extensor side. Reddening of the skin in general, and clearly visible, many distinct red spots which are being interconnected to a red patch-like pattern can be seen (Fig. 2, upper). Two weeks after treatment, general reddening of the skin had disappeared, showing again white, normal skin. The spots are reduced in number and reduced in their intensity, the interconnected patch-like pattern does not exist any more. (fig. 2, lower)

### Legends of figures:

Fig. 1: Normalization of irritated, atopic dermatitic skin of fingers after 1 week of application of microsilver cream (upper) and normalized skin after 3 weeks of application (lower) from example 6.
Fig. 2: Appearance of irritated skin of the crook of the arm (flexor side) after glucocorticoid treatment prior to application of microsilver cream (upper) and normalized skin after 2 weeks of application (lower) from example 7.

## Claims

1. Matrix formulation selected from a gel, crème, lotion or fluid, said formulation containing a water phase and a composition containing a combination of solid lipid particles in form of lipid nanoparticles and/or lipid microparticles with microsilver particles having a mean diameter of 1 to 100 *µ*m, and optionally oil droplets, wherein the microsilver particles are contained in the water phase of said matrix formulation.

2. Matrix formulation according to claim 1 wherein said microsilver particles in contact with water are able to release metal ions.

3. Matrix formulation according to any of claims 1 or 2, wherein said microsilver particles are solid or sponge-like.

4. Matrix formulation according to any of claims 1 to 3, containing additionally soluble silver salts, in particular silver nitrate, silver carbonate, silver sulphate, silver acetate, silver benzoate, silver lactate, organic silver compounds, in particular silver acetylacetonate, silver neodecanoate, silver ethylenediamintetraacetate, or any mixtures thereof.

5. Matrix formulation according to any of claims 1 to 4, wherein the microsilver particles have a mean diameter of 1 to 40 *µ*m, and preferentially 1 to 20 *µ*m.

6. Matrix formulation according to any of claims 1 to 5, wherein the lipid particles are lipid nanoparticles and posses a size below 1000 nm, especially 500 nm, preferably below 200 nm and more preferred in the range of 20 to 100 nm.

7. Matrix formulation according to any of claims 1 to 6, additionally containing pharmaceutical actives immune suppressive drugs (e.g. cyclosporine, tacrolimus, sirolimus) glucocorticoids (e.g. dehydrocortison, prednicarbate, dexamethasone, hydrocortison etc.), anti-virals, antimycotics (e.g. Amphotericin B, bifonazole, clotrimazole, itraconazole), antibiotics (e.g. tetracycline base), local anaesthetics (e.g. bupivacaine, tetracaine base), analgesics (e.g. diclofenac, ibuprofen, acetylsalicylic acid), anti-inflammatory drugs, cytotoxics (e.g. paclitaxel, 5-fluorouracil), aescin, minoxidil, hormones (e.g. estradiol, progesterone), caffeine, tretinoin, Sphingosin 1-Phosphat (S1P), alone or in any mixtures thereof.

8. Matrix formulation according to any of claims 1 to 7, wherein the concentration range of the microsilver particles is typically 0.00001% to 10%, preferably 0.00001% to 5%.

## Patentansprüche

1. Matrixformulierung, die aus Gel, Creme, Lotion oder Fluid ausgewählt ist, wobei die Formulierung eine Wasserphase und eine Zusammensetzung enthält, die eine Kombination von festen/flüssigen Teilchen in Form von Lipidnanoteilchen und/oder Lipidmikroteilchen mit Mikrosilberteilchen mit einem mittleren Durchmesser von 1 bis100 µm und gegebenenfalls Öltropfen enthält, wobei die Mikrosilberteilchen in der Wasserphase der Matrixformulierung enthalten sind.

2. Matrixformulierung nach Anspruch 1, bei der die Mikrosilberteilchen in Kontakt mit Wasser Metallionen freisetzen können.

3. Matrixformulierung nach einem der Ansprüche 1 oder 2, bei der die Mikrosilberteilchenfest oder schwammartig sind.

4. Matrixformulierung nach einem der Ansprüche 1 bis 3, die zusätzliche lösliche Silbersalze enthält, insbesondere Silbernitrat, Silbercarbonat, Silbersulfat, Silberacetat, Silberbenzoat, Silberlactat, organische Silberverbindungen, insbesondere Silberacetylacetonat, Silberneodecanoat, Silberethylendiamintetraacetat oder irgendwelche Mischungen derselben.

5. Matrixformulierung nach einem der Ansprüche 1 bis 4, bei der die Mikrosilberteilchen einen mittleren Durchmesser von 1 bis 40 µm und vorzugsweise 1 bis 20 µm aufweisen.

6. Matrixformulierung nach einem der Ansprüche 1 bis 5, bei der die Lipidteilchen Lipidnanoteilchen sind und eine Größe unter 1000 nm, insbesondere 500 nm, vorzugsweise unter 200 nm und bevorzugter im Bereich von 20 bis 100 nm aufweisen.

7. Matrixformulierung nach einem der Ansprüche 1 bis 6, die zusätzlich pharmazeutische Wirkstoffe, immunsuppressive Arzneistoffe (z.B. Cyclosporin, Tacrolimus, Sirolimus), Glucocorticoide (z.B. Dehydrocortison, Prednicarbate, Dexamethason, Hydrocortison usw.), antivirale Stoffe, Antimycotica (z.B. Amphotericin B, Bifonazol, Clotrimazol, Itraconazol), Antibiotica (z.B. Tetracyclinbase), Localanästhetica (z.B. Bupivacain, Tetracainbase), Analgetica (z.B. Diclofenac, Ibuprofen, Acetylsalicylsäure), entzündungshemmender Arzneistoffe, Cytotoxica (z.B. Paclitaxel, 5-Fluoruracil), Aescin, Minoxidil, Hormone (z.B. Östradiol, Progesteron), Koffein, Tretinoin, Sphingosin-1-Phosphat (S1 P), allein oder in irgendwelchen Mischungen derselben.

8. Matrixformulierung nach einem der Ansprüche 1 bis 7, bei der der Konzentrationsbereich der Mikrosilberteilchen typischerweise 0,00001% bis 10%, vorzugsweise 0,00001% bis 5% beträgt.

## Revendications

1. Formulation de matrice choisie parmi un gel, une crème, une lotion ou un fluide, ladite formulation contenant une phase aqueuse et une composition contenant un mélange de particules solides lipidiques sous forme de nanoparticules lipidiques et/ou de microparticules lipidiques avec des microparticules d'argent ayant un diamètre de 1 à 100 µm, et facultativement des gouttelettes d'huile, les microparticules d'argent étant contenues dans la phase aqueuse de ladite formulation de matrice.

2. Formulation de matrice selon la revendication 1, dans laquelle lesdites microparticules d'argent en contact avec l'eau sont capables de libérer des ions métalliques.

3. Formulation de matrice selon la revendication 1 ou 2, dans laquelle lesdites microparticules d'argent sont solides ou spongieuses.

4. Formulation de matrice selon la revendication 1 à 3, contenant en plus des sels d'argent solubles, en particulier du nitrate d'argent, du carbonate d'argent, du sulfate d'argent, de l'acétate d'argent, du benzoate d'argent, du lactate d'argent, des composés organiques de l'argent, en particulier de l'acétylacétonate d'argent, du néodécanoate d'argent, de l'éthylènediaminetétraacétate d'argent, et tout mélange de ceux-ci.

5. Formulation de matrice selon la revendication 1 à 4, dans laquelle les microparticules d'argent ont un diamètre moyen de 1 à 40 µm, et de préférence de 1 à 20 µm.

6. Formulation de matrice selon la revendication 1 à 5, dans laquelle les particules lipidiques sont des nanoparticules lipidiques et possèdent une taille inférieure à 1000 nm, en particulier 500 nm, de préférence inférieure à 200 nm et plus préférentiellement dans la gamme entre 20 et 100 nm.

7. Formulation de matrice selon la revendication 1 à 6, contenant en plus des médicaments pharmaceutiquement actifs immunosuppresseurs (ex cyclosporine, tacrolimus, sirolimus), des glucocorticoïdes (ex. déshydrocortisone, prednicarbate, dexaméthasone, hydrocortisone, etc.), des antiviraux, des antimycotiques (ex. amphotéricine B, bifonazole, clotrimazole, itraconazole), des antibiotiques (ex. tétracycline base), des anesthésiques locaux (ex. bupivacaïne, tétracaïne base), des analgésiques (ex. diclofénac, ibuprofène, acide acétylsalicylique), des anti-inflammatoires, des cytotoxiques (ex. paclitaxel, 5-fluorouracile), de l'aescine, du minoxidil, des hormones (oestradiol, progestérone), de la caféine, de la trétinoïne, de la sphingosine-1-phosphate (S1P), seul ou tout mélange de ceux-ci.

8. Formulation de matrice selon la revendication 1 à 7, dans laquelle la gamme de concentration des microparticules d'argent est typiquement de 0,00001 % à 10 %, de préférence de 0,00001 % à 5 %.
